# EUROPÄISCHE PATENTANMELDUNG

(11) **EP 1 464 295 A2**
(43) Veröffentlichungstag der Anmeldung: **06.10.2004**
(21) Anmeldenummer: 04002710.4
(22) Anmeldetag: 06.02.2004
(51) Int. Cl.: A61B 17/80, A61B 17/82

(54) **Implantat**

(30) Priorität: 01.04.2003 EP 03007543
(71) Anmelder: Centerpulse Orthopedics Ltd., 6340 Baar (CH)
(72) Erfinder: Schwammberger, Andy, 4434 Hölstein (CH); Velikov, Jordan, 8810 Horgen (CH)
(74) Vertreter: Manitz, Finsterwald & Partner GbR

(57) **Zusammenfassung**

Die Erfindung betrifft ein Implantat zur Versorgung von Knochenfrakturen, insbesondere von proximalen Humerusfrakturen, mit einer am Knochen fixierbaren Hauptplatte und wenigstens einem Ausleger, der über wenigstens ein flexibles Verbindungselement mit der Hauptplatte derart verbindbar ist, dass der Ausleger räumlich versetzt zur Hauptplatte am Knochen fixierbar ist.

## Beschreibung

Die Erfindung betrifft ein Implantat zur Versorgung von Knochenfrakturen mit einer am Knochen fixierbaren Hauptplatte.

Derartige Implantate, die auch als Osteosyntheseplatten bezeichnet werden, dienen beispielsweise zur Versorgung von proximalen Humerusfrakturen und sind grundsätzlich bekannt, beispielsweise aus der EP 0 468 192 B1.

Bestimmte Frakturen, insbesondere solche mit einer Mehrzahl von einzelnen Knochenfragmenten oder solche am Humerus, bei denen der Tuberculum minus mit betroffen ist, lassen sich mit derartigen Platten nicht optimal versorgen. Problematisch sind insbesondere solche Fälle, bei denen durch die Fraktur vergleichsweise weit auseinander liegende Bereiche des Knochens in Mitleidenschaft gezogen sind.

Aufgabe der Erfindung ist es, ausgehend von einem Implantat der eingangs genannten Art eine Möglichkeit zu schaffen, unterschiedlichste Frakturen sicher und zuverlässig zu versorgen, wobei es insbesondere möglich sein soll, auch komplizierte und vergleichsweise ausgedehnte Frakturen zufrieden stellend zu behandeln.

Die Lösung dieser Aufgabe erfolgt durch die Merkmale des Anspruchs 1 und insbesondere dadurch, dass das Implantat eine am Knochen fixierbare Hauptplatte und wenigstens einen Ausleger umfasst, der über wenigstens ein flexibles Verbindungselement mit der Hauptplatte derart verbindbar ist, dass der Ausleger räumlich versetzt zur Hauptplatte am Knochen fixierbar ist.

Mit dem erfindungsgemäßen Implantat können grundsätzlich beliebige räumliche Implantat-Strukturen realisiert und in einer gezielt an die jeweilige Fraktur angepassten Konfiguration an dem zu versorgenden Knochen fixiert werden. Dabei kann das Implantat wie ein den betroffenen Bereich umfassender Stabilisierungs- oder Haltekäfig oder wie eine den betroffenen Bereich umschließende Halte- oder Stabilisierungsklammer um den Knochen herum- bzw. an den Knochen angelegt werden. Die Flexibilität des Verbindungselementes gestattet dabei eine optimale Anpassung sowohl an die Form des Knochens als auch an den Verlauf der jeweiligen Fraktur. Mit dem bezüglich der Hauptplatte umfangsversetzt am Knochen fixierbaren Ausleger kann das erfindungsgemäße Implantat insgesamt eine sehr genaue Reposition der einzelnen Knochenfragmente erzielen und der Fraktur eine optimale Stabilität verleihen.

Vorteilhafte Ausführungsformen der Erfindung sind in den abhängigen Ansprüchen, der Beschreibung sowie der Zeichnung angegeben.

In einem bevorzugten Ausführungsbeispiel der Erfindung ist der Ausleger plattenförmig ausgebildet.

Des Weiteren kann vorgesehen sein, dass der Ausleger flexibel ausgebildet ist und insbesondere durch Verbiegen in eine jeweils erforderliche Raumform gebracht werden kann. Hierdurch kann nicht nur aufgrund des flexibel ausgestalteten Verbindungselementes die Position des Auslegers am Knochen relativ zu der Hauptplatte gezielt gewählt, sondern zusätzlich der Ausleger an die Anatomie des Knochens angepasst werden.

Ferner wird erfindungsgemäß vorgeschlagen, dass der Ausleger in die jeweils erforderliche Form und Größe zuschneidbar ist. Diese Anpassbarkeit der Form und der Größe des Auslegers an die jeweiligen Gegebenheiten ermöglicht es, ein für die jeweilige Fraktur maßgeschneidertes Implantat bereitzustellen.

Der Ausleger kann eine Mehrzahl von Durchgängen zur Aufnahme von Befestigungselementen aufweisen. Als Befestigungselemente kommen insbesondere Knochenschrauben in Frage. Die Befestigung des Auslegers am Knochen kann also grundsätzlich auf die gleiche Art und Weise erfolgen wie die Fixierung der Hauptplatte.

Der Ausleger kann insbesondere in Form einer Lochplatte vorgesehen sein.

In einem weiteren Ausführungsbeispiel der Erfindung ist der Ausleger netz- oder gitterförmig ausgebildet. Mit einer derartigen Netz- oder Gitterstruktur des Auslegers steht eine Vielzahl von Durchgängen oder Durchbrüchen im Ausleger zur Verfügung, durch welche zur Fixierung des Auslegers am Knochen dienende Befestigungselemente, insbesondere Knochenschrauben, hindurch geführt werden können. Der erfindungsgemäße Ausleger und damit das erfindungsgemäße Implantat sind hierdurch besonders flexibel und vielseitig einsetzbar.

Es kann vorgesehen sein, dass der Ausleger eine Mehrzahl von direkt oder durch Stege miteinander verbundenen, jeweils einen Durchgang begrenzenden Ringabschnitten umfasst.

Der Ausleger kann einstückig mit dem Verbindungselement ausgebildet sein. Alternativ oder zusätzlich ist es möglich, dass der Ausleger mit beispielsweise ösenartigen oder ringförmigen Befestigungsabschnitten versehen ist, die zur Koppelung mit dem Verbindungselement dienen.

In einem weiteren bevorzugten Ausführungsbeispiel der Erfindung ist vorgesehen, dass der räumliche Versatz zwischen der Hauptplatte und dem Ausleger individuell einstellbar ist, und zwar insbesondere mittels des Verbindungselementes.

Dies kann insbesondere dadurch erreicht werden, dass das Verbindungselement an der Hauptplatte und/oder am Ausleger in unterschiedlichen Positionen fixierbar ist.

In einem Ausführungsbeispiel der Erfindung kann die Hauptplatte wenigstens einen Durchgang zum Hindurchführen des Verbindungselementes aufweisen. Dabei kann sich der Durchgang im Wesentlichen parallel zu der durch die Hauptplatte festgelegten Plattenebene erstrecken.

Das Verbindungselement weist vorzugsweise eine langgestreckte Form auf. Ferner kann vorgesehen sein, dass das Verbindungselement verbiegbar ist. Bei dem Verbindungselement kann es sich insbesondere um einen Draht oder einen Faden handeln. Erfindungsgemäß kann das Verbindungselement sowohl plastisch als auch elastisch verformbar sein.

Die Koppelung des Verbindungselementes mit der Hauptplatte und/oder mit dem Ausleger kann durch Anbinden, Verhaken und/oder Verrasten erfolgen.

Die Anzahl der einzelnen Verbindungselemente zwischen der Hauptplatte und dem Ausleger ist grundsätzlich beliebig. Eine Anzahl von zwei sich etwa parallel erstreckenden Verbindungselementen hat sich sowohl hinsichtlich Handhabbarkeit als auch Genauigkeit und Stabilität als ausreichend erwiesen.

Erfindungsgemäß kann des Weiteren vorgesehen sein, dass wenigstens zwei jeweils durch zumindest einen Durchgang der Hauptplatte hindurch geführte Verbindungselemente auf der dem Ausleger abgewandten Seite der Hauptplatte miteinander verbindbar sind. Dieses Verbinden kann insbesondere durch Verknoten oder Zusammendrehen der freien Enden der Verbindungselemente erfolgen, wobei hierdurch insbesondere eine Zugspannung zwischen Ausleger und Hauptplatte erzeugt werden kann.

Der Ausleger und/oder das Verbindungselement sind insbesondere aus Metall, z.B. Titan, oder aus Kunststoff hergestellt. Bei den Kunststoffen kann es sich um bioresorbierbare Kunststoffe wie zum Beispiel um Lactate handeln. Wenn in einem solchen Fall auch noch bioresorbierbare Verbindungselemente, zum Beispiel bioresorbierbares Nahtmaterial, verwendet werden, kann die Explantation der Hauptplatte mit einem minimalinvasiven Eingriff erfolgen.

Ferner kann vorgesehen sein, dass die Hauptplatte und/ oder der Ausleger wenigstens einen haken- oder klauenartigen Fortsatz aufweist. Die Positionierung und Fixierung der Hauptplatte bzw. des Auslegers am Knochen kann durch einen derartigen Fortsatz erleichtert werden.

Des Weiteren ist erfindungsgemäß vorzugsweise vorgesehen, dass unterschiedliche Implantatkonfigurationen aus Hauptplatte und mit der Hauptplatte verbundenem Ausleger herstellbar sind, die bezüglich der Hauptplatte und insbesondere bezüglich einer Längsachse der Hauptplatte symmetrisch sind. So kann beispielsweise eine Links-/Rechts-Symmetrie des Implantats realisiert werden, die dessen Einsatzmöglichkeiten vergrößert.

Gemäß einem weiteren Ausführungsbeispiel der Erfindung kann vorgesehen sein, dass der Ausleger und das Verbindungselement separat hergestellt und derart fest miteinander verbunden sind, dass Ausleger und Verbindungselement während einer Operation als eine Einheit handhabbar sind. Hierdurch wird die Handhabung des erfindungsgemäßen Implantats während der Operation erheblich vereinfacht, wobei es jedoch nach wie vor grundsätzlich möglich ist, den räumlichen Versatz zwischen Hauptplatte und Ausleger dadurch individuell einzustellen, dass die Hauptplatte relativ zum Verbindungselement bewegt wird, bevor die endgültige Fixierung des Auslegers an der Hauptplatte erfolgt.

Vorzugsweise sind der Ausleger und das Verbindungselement unlösbar miteinander verbunden. Eine innige Verbindung zwischen Ausleger und Verbindungselement kann beispielsweise durch Verschweißen hergestellt werden. Es ist zwar möglich, jedoch nicht unbedingt erforderlich, dass der Ausleger und das Verbindungselement aus dem gleichen Material hergestellt sind. So kann beispielsweise der Ausleger aus Kunststoff und das Verbindungselement aus Metall hergestellt sein, oder umgekehrt.

Gemäß einem weiteren bevorzugten Ausführungsbeispiel der Erfindung sind die Hauptplatte und der Ausleger über das Verbindungselement einseitig miteinander verbunden. Beispielsweise von der Hauptplatte aus gesehen erstreckt sich das Verbindungselement folglich nur ausgehend von einer Seite der Hauptplatte zum Ausleger. Insbesondere bilden Hauptplatte, Ausleger und Verbindungselement im implantierten Zustand keine "geschlossene" Struktur, die den betreffenden Knochen vollumfänglich umgibt, d. h. ein "Umwickeln" des betreffenden Knochens durch das erfindungsgemäße Implantat erfolgt bei diesem Ausführungsbeispiel nicht. Vielmehr wird der Knochen von dem erfindungsgemäßen Implantat lediglich über einen Teil seines Umfangs "umgriffen". Dabei kann in Abhängigkeit von dem betreffenden Knochen und der zu versorgenden Fraktur die Länge des Verbindungselementes bzw. der Abstand zwischen Hauptplatte und Ausleger derart bemessen sein, dass Hauptplatte und Ausleger am Knochen nicht diametral einander gegenüberliegen.

Ferner wird erfindungsgemäß vorgeschlagen, dass der Ausleger plattenförmig ausgebildet ist und eine geringere Dicke aufweist als die Hauptplatte. Insbesondere kann die Dicke des Auslegers weniger als die Hälfte der Dicke der Hauptplatte betragen.

Der Ausleger kann ferner derart ausgebildet sein, dass er während einer Operation werkzeuglos verformbar ist. Hierdurch kann der Operateur während der Operation den Ausleger unmittelbar mit seinen Händen insbesondere durch Verbiegen derart verformen, dass der Ausleger unter Berücksichtigung seiner Sollposition optimal an die Kontur des Knochens angepasst wird.

Bei einem Ausleger aus bioresorbierbarem Material, beispielsweise einem Polymer, und bei bioresorbierbaren Verbindungselementen in Form von Fäden, wie es in einem bevorzugten Ausführungsbeispiel vorgesehen ist, erübrigt sich die spätere Explantation, wenn auch bioresorbierbare Knochenschrauben zur Verankerung verwendet werden.
Im Weiteren können bioresorbierbare Polymere für den Ausleger vorgesehen sein, die eine plastische Verformung von Hand zulassen, wenn sie in einem Salzbad, zum Beispiel in Ringer-Lösung, auf Temperaturen zwischen 50 und 90°C erwärmt wurden.

Des Weiteren wird erfindungsgemäß vorgeschlagen, dass der Ausleger eine kleinere Grundfläche aufweist als die Hauptplatte.

Außerdem kann erfindungsgemäß vorgesehen sein, dass der Ausleger eine für alle gängigen Frakturen eines bestimmten Knochens ausreichend große Grundform aufweist und zur Anpassung an eine jeweils zu versorgende Knochenfraktur in die erforderliche Form und Größe zuschneidbar ist.

Vorzugsweise ist der Ausleger mit wenigstens fünf Durchgängen zur Aufnahme von Befestigungselementen versehen, wobei die Befestigungselemente vorzugsweise in Form von Knochenschrauben vorgesehen sind.

Die Erfindung betrifft außerdem ein Implantatsystem zur Versorgung von Knochenfrakturen, insbesondere von proximalen Humerusfrakturen, mit wenigstens einer am Knochen fixierbaren Hauptplatte, wenigstens einem Ausleger und einem Satz von flexiblen Verbindungselementen, über welche der Ausleger mit der Hauptplatte derart verbindbar ist, dass der Ausleger räumlich versetzt zur Hauptplatte am Knochen fixierbar ist, wobei die Verbindungselemente einsatzbereit vorgefertigt sind und sich hinsichtlich Form, Größe und/oder Länge voneinander unterscheiden.

Hierbei kann der Operateur während der Operation aus einem Satz von vorgefertigten Verbindungselementen das jeweils passende Verbindungselement auswählen und ohne Zeitverlust ein optimal an die jeweilige Fraktur angepasstes Implantat zusammenstellen.

Eine mögliche Befestigung an der Hauptplatte kann darin bestehen, dass die vom Ausleger kommenden Drähte oder Fäden durch krimpen an der Hauptplatte befestigt werden.

Vorzugsweise ist vorgesehen, dass zumindest eines der Verbindungselemente eine U-Form aufweist und sowohl im Ausleger als auch in der Hauptplatte jeweils wenigstens ein Paar von insbesondere in Form von Bohrungen vorgesehenen Durchgängen für ein Verbindungselement ausgebildet ist, deren Abstand dem der U-Schenkel des Verbindungselementes entspricht.

Des Weiteren kann erfindungsgemäß ein Satz von Auslegen vorgesehen sein, die sich zumindest hinsichtlich der Anzahl von zur Aufnahme von Befestigungselementen insbesondere in Form von Knochenschrauben dienenden Durchgängen voneinander unterscheiden. Insbesondere unterscheiden sich die Ausleger außerdem hinsichtlich ihrer Größe und/oder Form. Hierdurch kann auf ein während der Operation durchzuführendes Zuschneiden von lediglich eine einzige Grundform bzw. -größe aufweisenden Auslegern verzichtet werden.

Das erfindungsgemäße Implantat aus Hauptplatte und Ausleger wird im Rahmen einer offenen Operation verwendet, d.h. minimal-invasive Techniken kommen in Verbindung mit dem erfindungsgemäßen Implantat nicht zum Einsatz.

Vorzugsweise ist aber die Hauptplatte derart ausgebildet, dass sie nicht nur in Verbindung mit dem erfindungsgemäßen Ausleger, sondern auch alleine verwendet werden kann. Zur Versorgung von Frakturen, bei denen der zusätzliche Ausleger nicht erwünscht oder erforderlich ist, kann die Hauptplatte dann auch im Rahmen minimal-invasiver Operation zum Einsatz kommen und dabei mit Hilfe eines mit der Hauptplatte lösbar verbundenen Handgriffs über einen kleinen Einschnitt in den Körper eingeführt und am Knochen positioniert werden. Zur Fixierung der. Hauptplatte am Knochen ist der Griff als Zielhilfe für Knochenschrauben ausgebildet bzw. wird der Griff durch eine derartige Zielhilfe ersetzt. Bei Nichtverwendung des erfindungsgemäßen Auslegers kann die Hauptplatte folglich so eingesetzt werden, wie es in der eingangs bereits erwähnten EP 0 468 192 B1 beschrieben ist.

Wenn das erfindungsgemäße Implantat aus Hauptplatte und Ausleger zum Einsatz kommt, dann kann grundsätzlich der Ausleger sowohl vor als auch nach Fixierung der Hauptplatte am Knochen über das Verbindungselement mit der Hauptplatte verbunden und anschließend ebenfalls am Knochen fixiert werden.

Die Erfindung wird im Folgenden beispielhaft unter Bezugnahme auf die Zeichnungen beschrieben. Es zeigen:
- Fig. 1: eine Ausführungsform eines erfindungsgemäßen Implantats im an einem Knochen fixierten Zustand,
- Fig. 2: eine weitere Ausführungsform eines erfindungsgemäßen Implantats,
- Fig. 3, 4, 5a-5c: verschiedene Ausführungsformen eines Auslegers eines erfindungsgemäßen Implantats,
- Fig. 6: verschiedene Möglichkeiten zur Koppelung von Verbindungselementen mit einer Hauptplatte eines erfindungsgemäßen Implantats,
- Fig. 7a-7f: weitere Möglichkeiten zur Koppelung eines Verbindungselementes an einer Hauptplatte eines erfindungsgemäßen Implantats bzw. spezielle Ausgestaltungen der Hauptplatte,
- Fig. 8a-8c: verschiedene Ansichten einer Hauptplatte gemäß einer weiteren Ausführungsform der Erfindung,
- Fig. 9: verschiedene Ansichten eines Auslegers gemäß einer weiteren Ausführungsform der Erfindung, und
- Fig. 10: verschiedene Ansichten eines Verbindungselementes gemäß einer weiteren Ausführungsform der Erfindung.

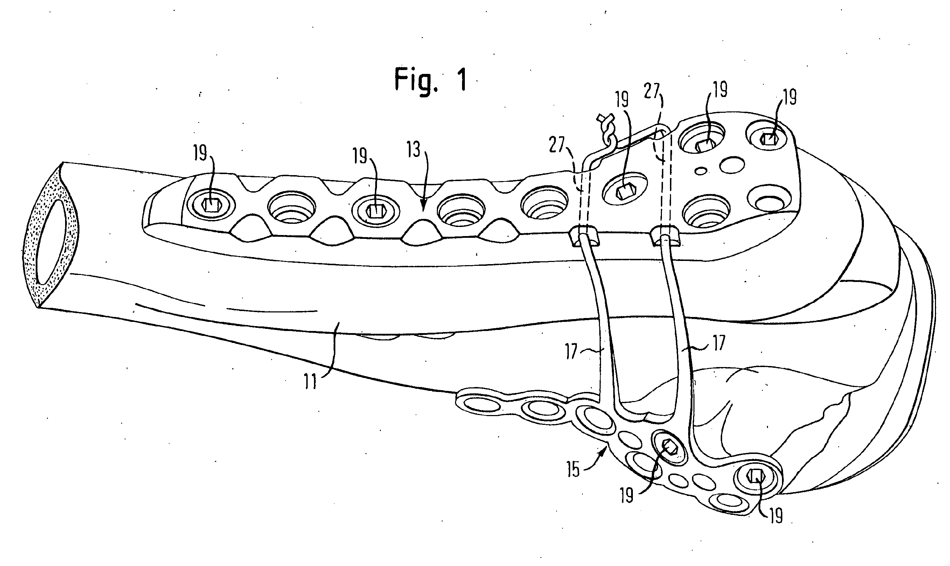

Fig. 1 zeigt ein erfindungsgemäßes Implantat aus einer Hauptplatte 13, einem plattenförmigen Ausleger 15 sowie zwei Verbindungselementen 17, das hier zur Versorgung des Humerus 11 eingesetzt wird, wobei der Ausleger 15 zur Befestigung an Knochenfragmenten, beim Humerus 11 zum Beispiel an einer Tuberculum minus-Fraktur dient. Die Auslegerplatte 15 erstreckt sich nicht in einer Ebene, sondern ist gezielt durch Verbiegen an die im für den Einsatz der Auslegerplatte 15 vorgesehenen Bereich gegebene Anatomie des Knochens 11 angepasst.

Sowohl die Hauptplatte 13 als auch die an den Knochen 11 angeformte Auslegerplatte 15 sind mittels Knochenschrauben 19 am Knochen 11 fixiert, die in Bohrungen der Hauptplatte 13 bzw. des Auslegers 15 aufgenommen sind.

Die beispielsweise aus Titan hergestellte Auslegerplatte 15 ist einstückig mit zwei langgestreckten, drahtartigen Verbindungselementen 17 ausgebildet, die jeweils durch einen in der Hauptplatte 13 ausgebildeten, sich etwa parallel zur Hauptplattenebene erstreckenden Durchgang 27 hindurch geführt sind. Die freien Enden der Verbindungselemente 17 sind durch Zusammendrehen miteinander verbunden, wodurch ein versehentliches Herausziehen der Verbindungselemente 17 aus der Hauptplatte 13 vermieden wird und ein maximaler Abstand zwischen der Hauptplatte 13 und der Auslegerplatte 15 vorgegeben ist.

Nicht nur die Auslegerplatte 15, sondern auch die Verbindungselemente 17 können durch Verbiegen in die jeweils gewünschte Form gebracht werden, wodurch stets gezielt die für die jeweilige Fraktur benötigte räumliche Struktur des Implantats realisiert werden kann.

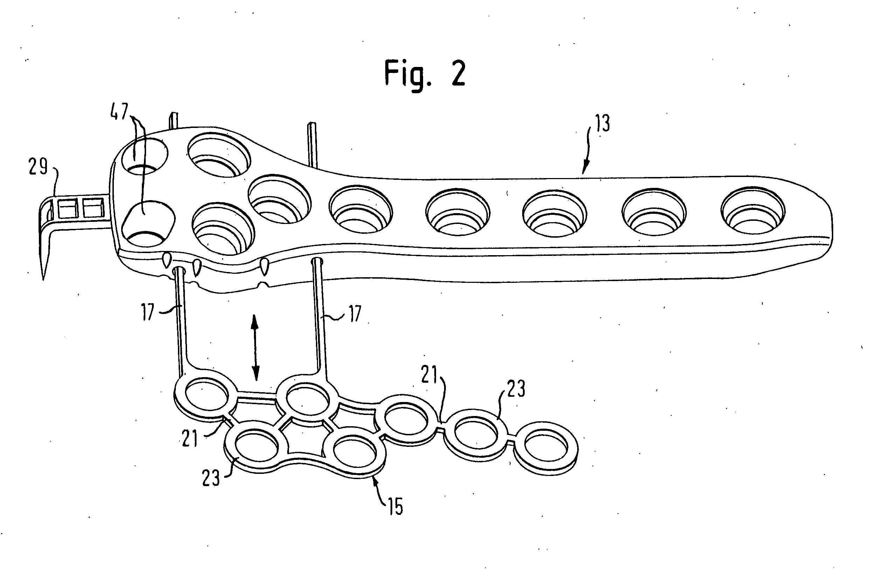

Fig. 2 zeigt ein Ausführungsbeispiel eines erfindungsgemäßen Implantats, bei dem die Hauptplatte 13 mit einem hakenförmigen Fortsatz 29 versehen ist, mit dem die Positionierung der Hauptplatte 13 am Knochen erleichtert wird.

Durch den Doppelpfeil in Fig. 2 ist angedeutet, dass der räumliche Versatz oder Abstand zwischen der Hauptplatte 13 und dem Ausleger 15 vor der endgültigen Fixierung der Verbindungselemente 17 an der Hauptplatte 13 verändert und auf diese Weise das erfindungsgemäße Implantat exakt an die jeweils zu versorgende Knochenfraktur angepasst werden kann.

Die Auslegerplatte 15 ist gitterförmig ausgebildet und umfasst eine Mehrzahl von Ringabschnitten 23, die durch Stege 21 miteinander verbunden sind, deren Länge kleiner als der Durchmesser der Ringabschnitte 23 ist. Jeder Ringabschnitt 23 definiert einen Durchgang, durch den eine Knochenschraube zur Fixierung des Auslegers 15 am zu versorgenden Knochen hindurch geführt werden kann.

Über die beiden vorderen, dem Haken 29 nächstgelegenen Bohrungen 47 der Hauptplatte 13 kann diese mit einem Griff und/oder mit einer Zielhilfe verbunden werden, wie er bzw. sie im Einleitungsteil erläutert wurde. Ein derartiger Griff bzw. eine derartige Zielhilfe ist nicht Gegenstand der Erfindung, so dass hierauf im Folgenden nicht näher eingegangen wird.

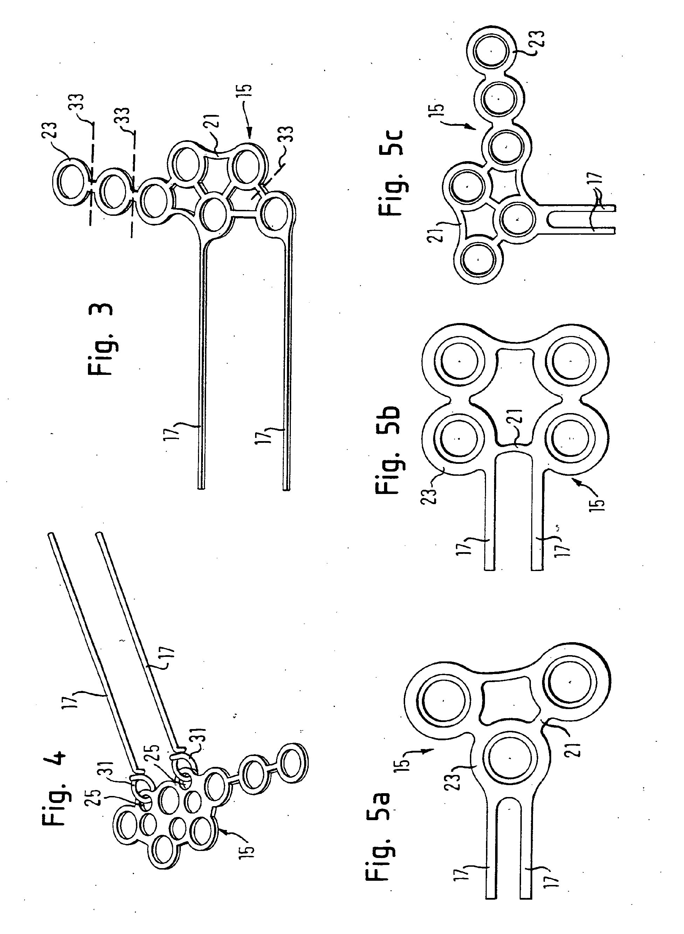

Die in den Fig. 3 und 4 gezeigten Ausführungsformen einer erfindungsgemäßen Auslegerplatte 15 unterscheiden sich voneinander zum einen dadurch, dass bei der Variante gemäß Fig. 4 Bohrungen in einem mittleren Bereich in einem vollflächigen Material ausgebildet sind, der Ausleger 15 also in Form einer Lochplatte vorgesehen ist, während bei der Variante gemäß Fig. 3 einzelne Ringabschnitte 23 entweder direkt oder über Stege 21 miteinander verbunden sind, wodurch eine zusammenhängende, gitter- oder netzartige Lochstruktur erhalten wird.

Zum anderen unterscheiden sich diese beiden Varianten voneinander durch die Art und Weise der Koppelung der Verbindungselemente an den Ausleger 15. In der Variante gemäß Fig. 4 sind die Verbindungselemente 17 jeweils an ihrem einen freien Ende zu Ösen 31 gebogen, die in als Befestigungsabschnitte dienende Bohrungen 25 der Auslegerplatte 15 eingehakt sind. In der Variante gemäß Fig. 3 sind die Verbindungselemente 17 einstückig mit der Auslegerplatte 15 ausgebildet.

Durch die gestrichelten Linien in Fig. 3 sind beispielhaft mögliche Schnittlinien 33 angedeutet, entlang welcher die Auslegerplatte 15 durch Wegnehmen eines oder mehrerer Ringabschnitte 23 auf die jeweils benötigte Größe zurechtgeschnitten werden kann.

Die Fig. 5a-5c zeigen beispielhaft, wie durch eine unterschiedliche Anzahl von Ringelementen 23 und eine unterschiedliche Art und Weise der Verbindung der Ringabschnitte 23 zu einer zusammenhängenden Auslegerplatte 15 verschiedene Formen und Größen der Auslegerplatte 15 realisiert werden können.

Wie in der Variante gemäß Fig. 3 sind in den Beispielen der Fig. 5a-5c die Verbindungselemente 17 einstückig mit der Auslegerplatte 15 verbunden, wobei entweder beide Verbindungselemente 17 von dem gleichen Ringabschnitt 23 (vgl. Fig. 5a) ausgehen oder mit verschiedenen Ringabschnitten 23 (vgl. Fig. 5b und 5c) verbunden sein können.

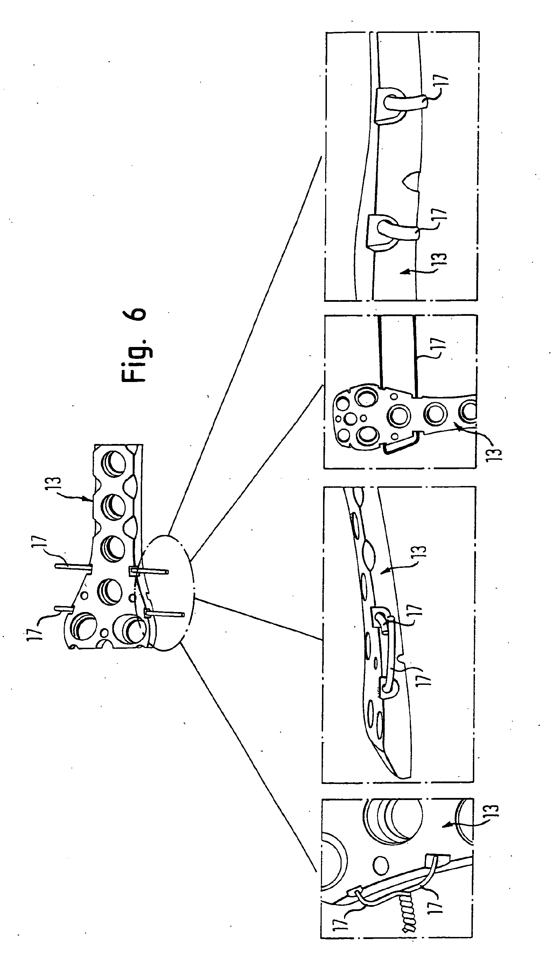

Wie Fig. 6 anhand von vier Beispielen zeigt, kann die Koppelung der hier jeweils als biegsamer Draht ausgebildeten Verbindungselemente 17 an die Hauptplatte 13 (von links nach rechts) (i) durch Zusammendrehen der freien Enden zweier durch die Hauptplatte 13 hindurch gesteckter Verbindungselemente 17 (vgl. auch Fig. 1), (ii) durch Umbiegen der aus der Hauptplatte 13 heraus ragenden freien Enden der Verbindungselemente 17 in der Ebene der Hauptplatte 13, (iii) durch Hindurchführen lediglich eines einzigen Verbindungselementes 17 durch einen in der Hauptplatte 13 ausgebildeten Durchgang in der einen Richtung und durch Zurückführen dieses Verbindungselementes 17 durch einen weiteren in der Hauptplatte 13 ausgebildeten Durchgang in der anderen Richtung, oder (iv) durch Umbiegen der aus der Hauptplatte 13 heraus ragenden freien Enden der Verbindungselemente 17 senkrecht zur Ebene der Hauptplatte 13 erfolgen.

Allen gezeigten Varianten der Fig. 6 ist gemeinsam, dass ein versehentliches Herausziehen der Verbindungselemente 17 aus der Hauptplatte 13 und damit ein Lösen der Verbindung zwischen Hauptplatte 13 und in Fig. 6 nicht dargestellter Auslegerplatte 15 vermieden wird.

Die Verbindung der Hauptplatte 13 mit der Auslegerplatte 15 über die Verbindungselemente 17 kann vor oder während der Operation erfolgen. Durch entsprechende Wahl der Länge der Verbindungselemente 17 kann der räumliche Versatz zwischen Hauptplatte 13 und Auslegerplatte 15 individuell an die jeweils zu versorgende Knochenfraktur angepasst werden.

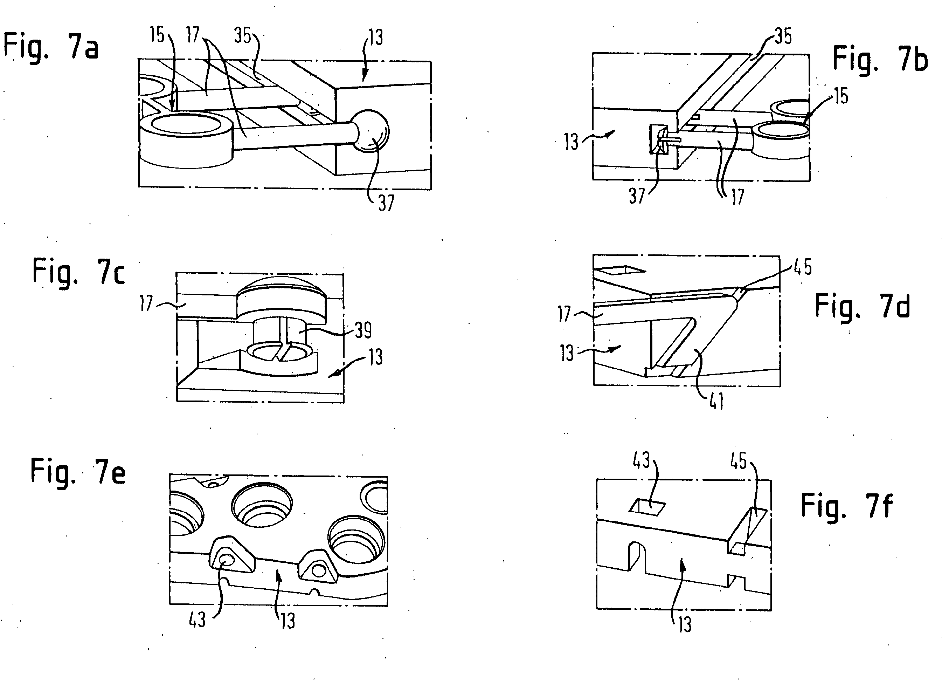

Die Fig. 7a-7d zeigen weitere Beispiele für die Koppelung der Verbindungselemente 17 an die Hauptplatte 13.

Gemäß Fig. 7a und 7b kann die Hauptplatte 13 mit Kanälen 35 versehen sein, die sich bodenseitig erweitern und so eine Hinterschneidung bilden, in die entsprechend geformte, beispielsweise kugelförmig erweiterte freie Enden 37 der Verbindungselemente 17 durch seitliches Hineinstecken (Fig. 7a) oder durch Einklipsen (Fig. 7b) herausziehsicher eingeführt werden können. Bei diesen beiden Varianten besteht in vorteilhafter Weise die Möglichkeit einer Verschiebbarkeit der Auslegerplatte 15 relativ zur Hauptplatte 13 senkrecht zur Längserstreckung der Verbindungselemente 17.

In der Variante gemäß Fig. 7c sind die freien Enden der Verbindungselemente 17 mit Befestigungsstiften 39 versehen, die an entsprechend ausgebildeten Aussparungen der Hauptplatte 13 mit dieser verrastet werden können.

In der Variante gemäß Fig. 7d sind die freien Enden 41 der Verbindungselemente 17 hakenförmig ausgebildet und an entsprechend ausgebildeten, jeweils als Befestigungsabschnitt dienenden Aussparungen 45 in die Hauptplatte 13 eingehakt.

Wie die Fig. 7e und 7f zeigen, kann die Hauptplatte 13 zusätzlich mit Durchführungen 43 versehen sein, durch die Nähnadeln bzw. Nahtmaterial hindurchgeführt werden kann. Wenn solche Durchführungen schräg von der oberen Fläche in eine Seitenfläche der Hauptplatte 13 verlaufen, dann kann auch bei am Knochen fixierter Hauptplatte Nahtmaterial z.B. mittels einer Rundnadel eingezogen werden.

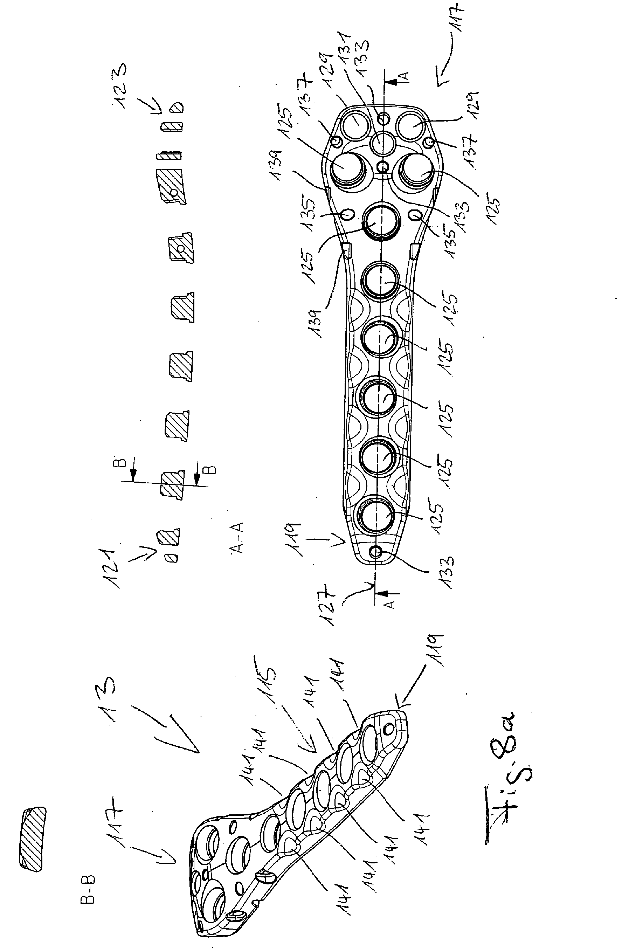

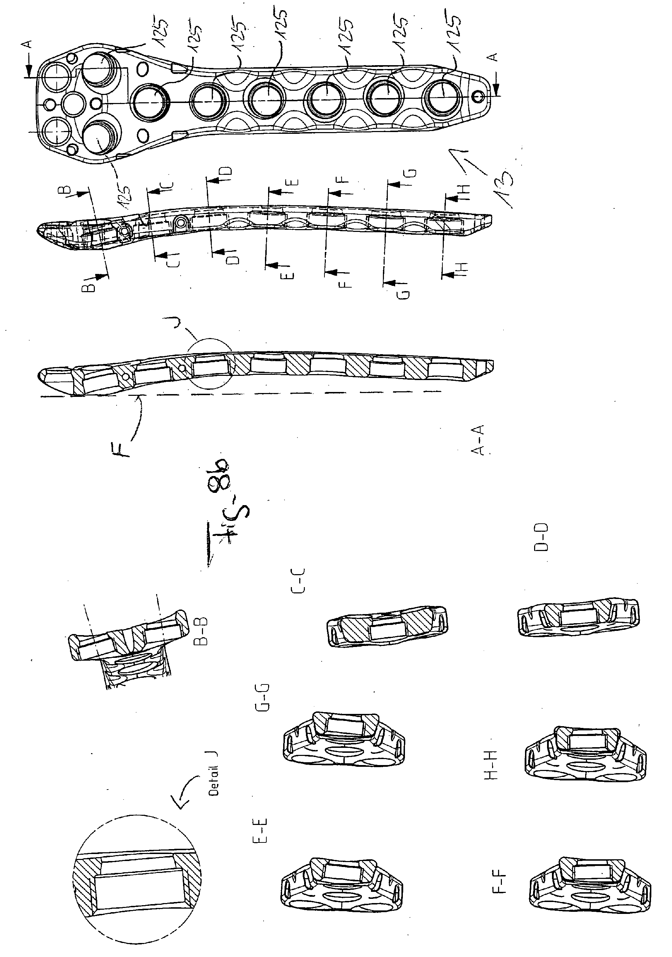

Die Fig. 8a, 8b und 8c zeigen verschiedene Ansichten einer bevorzugten Ausführungsform einer erfindungsgemäßen Hauptplatte 13.

Die Hauptplatte 13 kann in Verbindung mit Auslegerplatten sowie Verbindungselementen eingesetzt werden, auf die nachstehend näher eingegangen wird. Alternativ kann die Hauptplatte 13 aber auch alleine als Knochenplatte ohne Ausleger Verwendung finden.

Wie insbesondere die Draufsicht in Fig. 8a zeigt, besitzt die Hauptplatte 13 einen sich etwa über zwei Drittel ihrer Länge erstreckenden, relativ schmalen Abschnitt 115, an den sich ein verbreiterter Kopfabschnitt 117 anschließt, der das weitere Drittel der Plattenlänge bildet. Am anderen Ende des schmalen Abschnitts 115 schließt sich ein vergleichsweise kurzer, sich stetig etwa auf die Hälfte der Breite des schmalen Abschnitts 115 verjüngender Endabschnitt 119 an.

Insbesondere aus dem Schnitt A-A in Fig. 8a geht hervor, dass die Hauptplatte 13 nicht eben, sondern in Richtung ihrer Längserstreckung leicht gekrümmt derart ausgebildet ist, dass ein sanft geschwungener, wellenartiger Verlauf gegeben ist. Ausgehend etwa von der Plattenmitte verläuft die Hauptplatte 13 nach hinten unter einem relativ kleinen, wenige Grad betragenden Neigungswinkel schräg nach oben, wobei die Plattendicke bis zu einer hinteren Abschrägung 121 am kurzen Endabschnitt 119 ungefähr konstant ist und insbesondere im Bereich von etwa 4 bis 6 mm liegt. Ganz am Ende des abgeschrägten Endabschnitts 119 beträgt die Plattendicke weniger als die Hälfte der Dicke im schmalen Abschnitt 115.

Wiederum ausgehend etwa von der Plattenmitte verläuft die Hauptplatte 13 nach vorne ebenfalls unter einem relativ kleinen Winkel schräg nach oben, so dass der Kopfabschnitt 117 bezüglich der noch zum schmalen Abschnitt 115 gehörenden Plattenmitte angehoben ist, wobei jedoch - anders als im hinteren Bereich - der Kopfabschnitt 117 etwa parallel zum mittleren Teil der Hauptplatte 13 ausläuft. Am vorderen Ende ist der Kopfabschnitt 117 mit einer Abschrägung 123 versehen, so dass der Kopfabschnitt 117 genauso wie das Plattenende keilförmig ausläuft.

Die Übergänge zwischen den einzelnen Abschnitten der Platte 13 sind fließend ausgebildet, d. h. die Platte 13 weist bezüglich ihrer Längserstreckung eine relativ sanft gekrümmte Außenkontur ohne Kanten auf.

Wie insbesondere der Draufsicht in Fig. 8a zu entnehmen ist, ist die Platte 13 mit einer Vielzahl von Durchgängen in Form von Bohrungen versehen. Insgesamt acht Bohrungen 125 mit relativ großem Durchmesser dienen zur Aufnahme von Befestigungselementen in Form von Knochenschrauben. Die großen Bohrungen 125 sind jeweils mit einem Innengewinde versehen. Sechs große Bohrungen 125 sind in einer Reihe hintereinander zumindest im Wesentlichen gleich beabstandet längs der Mittelebene 127 der Platte 13 über den schmalen Abschnitt 115 bis in den Übergangsbereich zum Kopfabschnitt 117 verteilt angeordnet.

Die Achsen dieser großen Bohrungen 125 verlaufen bezüglich der Mittelebene 127 und/oder bezüglich einer Normalen auf einer Bezugsfläche F der Platte 13 geneigt. Dies gilt auch für die zwei großen Bohrungen 125 im Kopfabschnitt 117, die symmetrisch auf beiden Seiten der Mittelebene 127 einander gegenüber liegend angeordnet sind.

Die Hauptplatte 13 ist ferner mit drei weiteren Bohrungen 129, 131 von - relativ gesehen - mittlerer Durchmessergröße versehen. Die beiden am vorderen Ende des Kopfabschnitts 117 im Bereich der Abschrägung 123 symmetrisch zur Mittelebene 127 gelegenen Bohrungen 129 dienen zur Kopplung der Platte 13 mit einem Griff und/oder mit einer Zielhilfe, wie es im Einleitungsteil bereits erläutert wurde. Hierauf soll an dieser Stelle nicht näher eingegangen werden.

Ferner ist die Platte 13 mit drei auf der Mittelebene 127 liegenden kleinen Bohrungen 133 versehen, von denen eine auf der hinteren Abschrägung 121, eine auf der vorderen Abschrägung 123 und eine etwa zwischen den im Kopfabschnitt 117 ausgebildeten großen Bohrungen 125 auf der anderen Seite der mittelgroßen Bohrung 131 gelegen ist.

Auf am Kopfabschnitt 117 im Randbereich ausgebildete Randbohrungen 135, 137 sowie auf senkrecht zur Mittelebene 127 verlaufende Querbohrungen 139 wird an anderer Stelle näher eingegangen.

Die als Gewindebohrung ausgeführte Bohrung 125 kann im Zusammenwirken mit einer angrenzenden Bohrung 133 für die zeitweise Befestigung eines Handgriffs (nicht gezeigt) verwendet werden. Die Bohrungen 133, 135, 137 können als Befestigungshilfen für Spick-Drähte oder für Fäden, welche Weichteile wie Muskelenden oder Sehnen stützen, verwendet werden. Solche Fäden werden dann aus bioresorbierbarem Material verwendet, wenn die Weichteile sich später selbst genügend verankern können.

Zwischen den längs des schmalen Abschnitts 115 angeordneten großen Bohrungen 125 sind im Randbereich der Platte 13 auf jeder Seite jeweils vier muldenartige Vertiefungen 141 ausgebildet.

Wie insbesondere der Schnitt A-A in Fig. 8a und die verschiedenen Schnittansichten in Fig. 8b zeigen, sind die großen Bohrungen 125 stufenförmig ausgebildet. Ein von der Plattenoberseite ausgehender großer Teilbereich der Bohrungen 125 weist jeweils einen größeren Durchmesser auf und erstreckt sich über eine größere axiale Länge als ein kleiner Teilbereich der Bohrungen 125, der an der Plattenunterseite mündet. Das Innengewinde ist jeweils am großen oberen Teilbereich der Bohrungen 125 ausgebildet.

Die Schnitte B-B bis H-H der einzelnen großen Bohrungen 125 (Nr. 1 bis Nr. 8) sowie der Schnitt A-A in Fig. 8b zeigen, dass mit Ausnahme der Bohrungen Nr. 3 (Schnitt C-C) und Nr. 4 (Schnitt D-D) die Mittelachsen der Bohrungen 125 sowohl bezüglich einer Normalen auf der Bezugsfläche F (vgl. Schnitt A-A in Fig. 8b) als auch bezüglich der Mittelebene 127 der Platte 13 geneigt verlaufen. Die Mittelachsen der Bohrungen 125 mit den Nr. 5 bis 8 sind gegenüber der Mittelebene 127 um etwa 4° geneigt, während-dieser Neigungswinkel bei den im Kopfabschnitt 117 ausgebildeten Bohrungen 125 mit den Nr. 1 und 2 etwas geringer ist und vorzugsweise etwa 3,5° beträgt.

Die Mittelachsen der Bohrungen 125 mit den Nr. 3 (Schnitt C-C) und Nr. 4 (Schnitt D-D) liegen in der Mittelebene 127, sind jedoch bezüglich der erwähnten Normalen auf der Bezugsfläche F geneigt.

Fig. 8c zeigt insbesondere die Ausgestaltung der Randbohrungen 135, 137 sowie der Querbohrungen 139.

Wie aus den Schnitten A-A und B-B in Fig. 8c hervorgeht, sind die im hinteren Bereich des Kopfabschnitts 117 ausgebildeten Randbohrungen 135 (Schnitt B-B) stärker gegenüber der Normalen auf der Bezugsfläche F geneigt als die im vorderen Bereich des Kopfabschnitts 117 ausgebildeten Randbohrungen 137 (Schnitt A-A).

Die Querbohrungen 139 verlaufen jeweils senkrecht zur Mittelebene 127 der Platte 13 und besitzen einen vergleichsweise kleinen Innendurchmesser, der sich jeweils zur Mündung an den Schmalseiten der Platte 13 erweitert, wie insbesondere die Details E und F in Fig. 8c zeigen. Im Bereich der Mündungen verlaufen die Schmalseiten der Platte 13 schräg zur Längsachse der Querbohrungen 139.

Während die Querbohrungen 139 zur Anbindung einer oder mehrerer Ausleger mittels insbesondere in Form von Drähten vorgesehenen Verbindungselementen dienen, sind die Randbohrungen 135, 137 sowie die im Bereich der vorderen Abschrägung 123 ausgebildete Bohrung 133 zum Hindurchziehen von Nahtmaterial insbesondere unter Zuhilfenahme von gekrümmten Nadeln vorgesehen.

Nicht nur die Querbohrungen 139, sondern auch die schräg verlaufenden Randbohrungen 135, 137 erlauben ein Hindurchführen von Verbindungsdrähten bei am Knochen anliegender Hauptplatte 13, wodurch die Handhabung des Implantats insgesamt wesentlich verbessert wird.

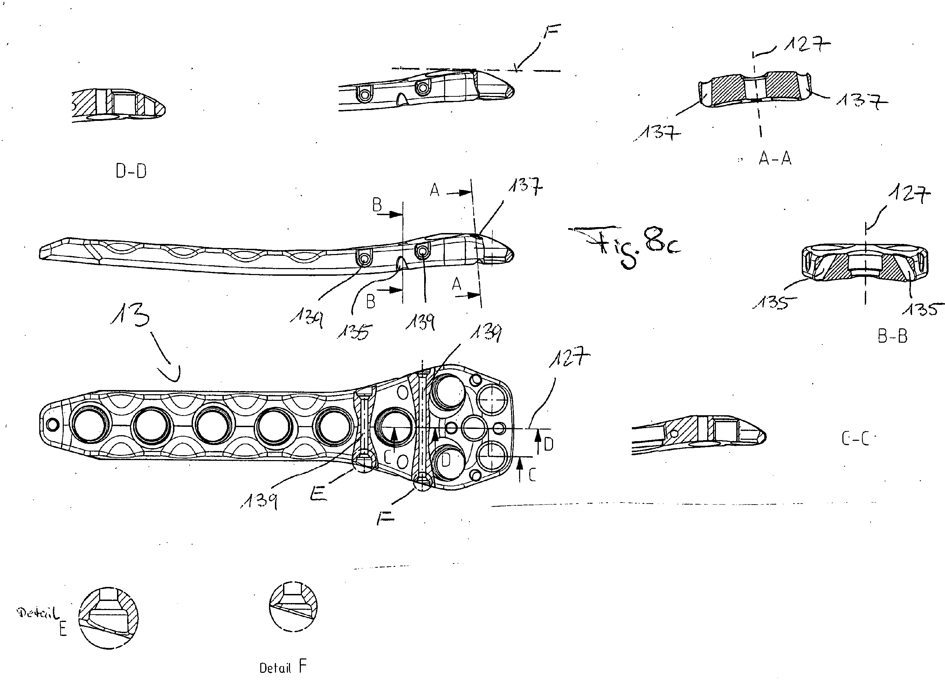

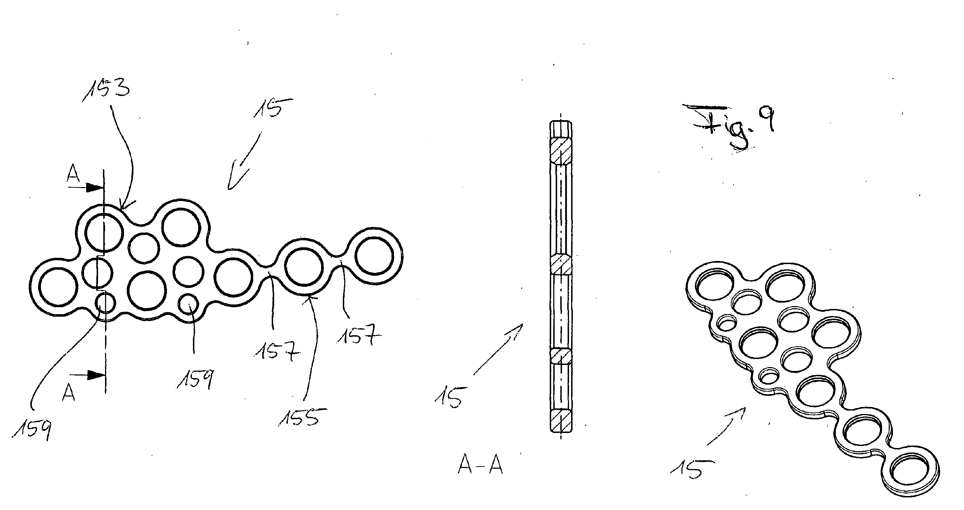

Fig. 9 zeigt ein bevorzugtes Ausführungsbeispiel eines im Folgenden auch als Auslegerplatte bezeichneten Auslegers 15. Der Ausleger 15 umfasst einen zusammenhängenden Lochplattenabschnitt 153, in welchem unterschiedlich große Bohrungen scheinbar ungeordnet ausgebildet sind, sowie einen Ringreihenabschnitt 155 aus drei geradlinig hintereinander angeordneten, durch Stege 157 miteinander verbundenen Ringabschnitten, die jeweils eine Bohrung begrenzen.

Die Außenkontur des Auslegers 15 folgt den Begrenzungen der Bohrungen derart, dass der Abstand des Plattenrandes zu der nächstgelegenen Bohrung oder zu einem gegenüberliegenden Bereich des Plattenrandes im Wesentlichen überall kleiner ist als der Durchmesser der kleinsten Sorte von Bohrungen, d.h. der Ausleger 15 wird gewissermaßen von einem relativ schmalen, wellenförmigen, umlaufenden Materialstreifen begrenzt.

In dem dargestellten Ausführungsbeispiel umfasst der Ausleger 15 drei Sorten von Bohrungen. Große Bohrungen, mit denen der Ringreihenabschnitt 155 ausschließlich versehen ist, besitzen einen Durchmesser von etwa 5 mm, wohingegen die mittleren Bohrungen einen Durchmesser von etwa 4 mm und die kleinen Bohrungen 159 einen Durchmesser von etwa 2,5 mm aufweisen.

Wie insbesondere aus dem Schnitt A-A in Fig. 9 hervorgeht, ist im Gegensatz zu der Hauptplatte 13 (vgl. Fig. 8a-8c) die Auslegerplatte 15 eben ausgeführt. Die Auslegerplatte 15 ist dabei wesentlich dünner als die Hauptplatte 13. Vorzugsweise beträgt die Dicke der Auslegerplatte 15 etwa 1 bis 2 mm, insbesondere etwa 1,2 mm.

Des Weiteren ist die Grundfläche der Auslegerplatte 15 kleiner als diejenige der Hauptplatte 13. Während die Auslegerplatte 15 vorzugsweise eine maximale Breite im Bereich von 15 bis 20 mm, vorzugsweise etwa 17,2 mm, und eine maximale Länge im Bereich von 50 bis 55 mm, vorzugsweise etwa 52,1 mm, aufweist, beträgt bevorzugt bei der Hauptplatte 13 die maximale Breite 22 bis 27 mm, vorzugsweise etwa 24,4 mm, und die maximale Länge 90 bis 95 mm, vorzugsweise etwa 93 mm.

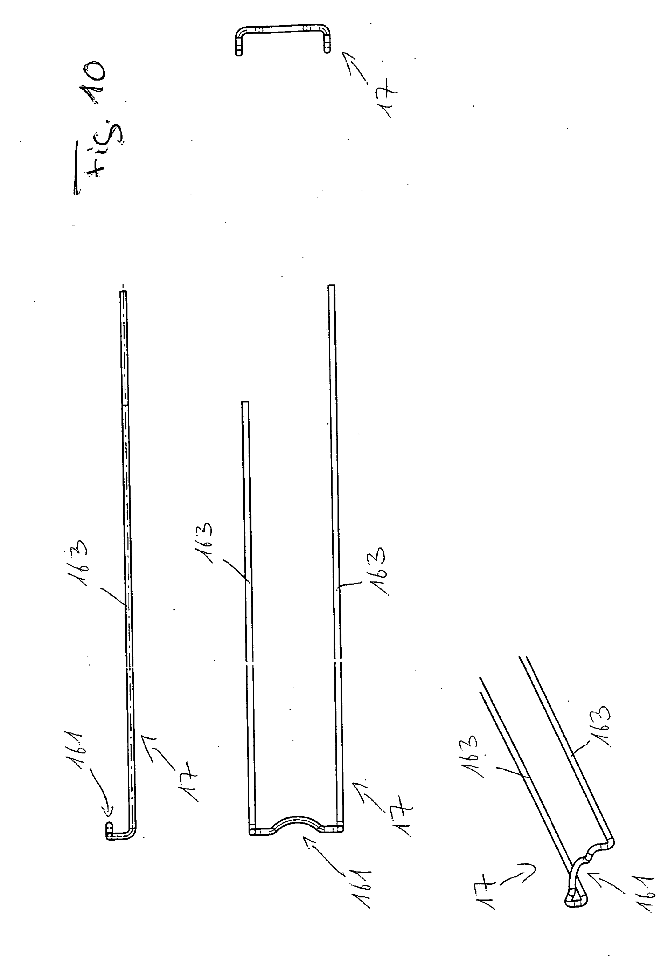

Fig. 10 zeigt verschiedene Ansichten eines als Verbindungselement 17 dienenden Cerclagedrahts.

Der Draht 17 ist in eine U-Form gebogen, die im Bereich des U-Stegs 161 rechtwinklig umgebogen ist, wobei im mittleren Bereich des umgebogenen U-Stegs 161 eine wiederum U-förmige Ausbuchtung vorgesehen ist.

Der Abstand zwischen den beiden U-Schenkeln 163 des Drahtes 17 entspricht dem Abstand der beiden kleinen Bohrungen 159 in der Auslegerplatte 15 (vgl. Fig. 9). Hierdurch kann der vorgefertigte Draht 17 problemlos durch die beiden Bohrungen 159 gesteckt und derart relativ zur Auslegerplatte 15 ausgerichtet werden, dass die beiden U-Schenkel 163 parallel zur Ebene der Auslegerplatte 15 verlaufen, ohne dass der U-Steg 161 über die zwischen den beiden kleinen Bohrungen 159 befindliche große Bohrung der Auslegerplatte 15 hinweg verläuft.

Die auf diese Weise von der mit dem Draht 17 gekoppelten Auslegerplatte 15 abstehenden U-Schenkel 163 des Drahtes 17 können anschließend in der jeweils erforderlichen Art und Weise verformt und mit der Hauptplatte 13 verbunden werden, indem sie mit ihren freien Enden durch die ebenfalls den entsprechenden Abstand aufweisenden Querbohrungen 139 gesteckt und an der gegenüberliegenden Seite der Hauptplatte 13 beispielsweise durch Umbiegen oder Zusammendrehen derart verriegelt werden, dass anschließend der Draht 17 und damit der Ausleger 15 herausziehsicher mit der Hauptplatte 13 verbunden ist.

Der Ausleger 15 und der Draht 17, die als separate Bauteile hergestellt sind, können vor der Operation und insbesondere bereits im Rahmen der Herstellung z. B. durch Verschweißen derart fest miteinander verbunden werden, dass sie während der Operation als eine Einheit handhabbar sind.

In dem vorstehend beschriebenen Ausführungsbeispiel, in welchem die Hauptplatte 13 lediglich ein Paar von Querbohrungen 139 aufweist, wird bevorzugt lediglich ein einziger Ausleger 15 über einen U-förmig vorgefertigten Draht 17 z.B. entsprechend Fig. 10 mit der Hauptplatte 13 verbunden. Die Hauptplatte 13 und der Ausleger 15 sind also lediglich einseitig mittels des Drahtes 17 miteinander verbunden, d.h. Draht 17 erstreckt sich nur ausgehend von einer Seite der Hauptplatte 13 zur Auslegerplatte 15.

Die insbesondere aus Titan bestehende Auslegerplatte 15 ist aufgrund ihrer geringen Dicke während der Operation werkzeuglos verformbar, so dass der Operateur allein unter Zuhilfenahme seiner Hände die im Ausgangszustand ebene Auslegerplatte 15 in die jeweils gewünschte Form bringen kann.

Die Auslegerplatte 15, die in dem Ausführungsbeispiel der Fig. 9 den gewissermaßen einen "Haufen" von Bohrungen umfassenden Lochplattenabschnitt 153 und den im Gegensatz dazu schmalen, langgestreckten Ringreihenabschnitt 155 umfasst, ist hinsichtlich ihrer Grundform derart gewählt, dass sie für alle gängigen Frakturen am Tuberculum minus ausreichend groß ist und durch Zuschneiden mit einem entsprechenden Werkzeug an die jeweils zu versorgende Knochenfraktur angepasst werden kann.

### Bezugszeichenliste

- 11: Knochen
- 13: Hauptplatte
- 15: Ausleger
- 17: Verbindungselement
- 19: Befestigungselement, Knochenschraube
- 21: Steg
- 23: Ringabschnitt
- 25: Befestigungsabschnitt, Bohrung
- 27: Durchgang
- 29: Fortsatz
- 31: Öse
- 33: Schnittlinie
- 35: Kanal
- 37: Erweiterung
- 39: Befestigungsstift
- 41: hakenförmiges Ende
- 43: Durchführung
- 45: Befestigungsabschnitt, Aussparung
- 47: Bohrung für Griff bzw. Zielhilfe
- 115: schmaler Abschnitt
- 117: Kopfabschnitt
- 119: Endabschnitt
- 121: hintere Abschrägung
- 123: vordere Abschrägung
- 125: große Bohrung
- 127: Mittelebene
- 129: Bohrung
- 131: Bohrung
- 133: Bohrung
- 135: Randbohrung
- 137: Randbohrung
- 139: Querbohrung
- 141: Vertiefung
- 153: Lochplattenabschnitt
- 155: Ringreihenabschnitt
- 157: Steg
- 159: kleine Bohrung des Auslegers
- 161: U-Steg
- 163: U-Schenkel

- F: Bezugsfläche der Hauptplatte

## Patentansprüche

1. Implantat zur Versorgung von Knochenfrakturen, insbesondere von proximalen Humerusfrakturen, mit einer am Knochen (11) fixierbaren Hauptplatte (13) und wenigstens einem Ausleger (15), der über wenigstens ein flexibles Verbindungselement (17) mit der Hauptplatte (13) derart verbindbar ist, dass der Ausleger (15) räumlich versetzt zur Hauptplatte (13) am Knochen (11) fixierbar ist.

2. Implantat nach Anspruch 1,
**dadurch gekennzeichnet,**
**dass** der Ausleger (15) plattenförmig ausgebildet ist.

3. Implantat nach Anspruch 1 oder 2,
**dadurch gekennzeichnet,**
**dass** der Ausleger (15) flexibel ausgebildet und insbesondere durch Verbiegen in eine jeweils erforderliche Raumform bringbar ist.

4. Implantat nach einem der vorhergehenden Ansprüche,
**dadurch gekennzeichnet,**
**dass** der Ausleger (15) in die jeweils erforderliche Form und Größe zuschneidbar ist.

5. Implantat nach einem der vorhergehenden Ansprüche,
**dadurch gekennzeichnet,**
**dass** der Ausleger (15) eine Mehrzahl von Durchgängen zur Aufnahme von Befestigungselementen (19), insbesondere von Knochenschrauben, aufweist.

6. Implantat nach einem der vorhergehenden Ansprüche,
**dadurch gekennzeichnet,**
**dass** der Ausleger (15) als Lochplatte ausgebildet ist.

7. Implantat nach einem der vorhergehenden Ansprüche,
**dadurch gekennzeichnet,**
**dass** der Ausleger (15) netz- oder gitterförmig ausgebildet ist.

8. Implantat nach einem der vorhergehenden Ansprüche,
**dadurch gekennzeichnet,**
**dass** der Ausleger (15) eine Mehrzahl von direkt oder durch Stege (21) miteinander verbundenen, jeweils einen Durchgang begrenzenden Ringabschnitten (23) umfasst.

9. Implantat nach einem der vorhergehenden Ansprüche,
**dadurch gekennzeichnet,**
**dass** der Ausleger (15) einstückig mit dem Verbindungselement (17) ausgebildet ist.

10. Implantat nach einem der vorhergehenden Ansprüche,
**dadurch gekennzeichnet,**
**dass** der Ausleger (15) insbesondere ösenartige oder ringförmige Befestigungsabschnitte (25) zur Koppelung mit dem Verbindungselement (17) aufweist.

11. Implantat nach einem der vorhergehenden Ansprüche,
**dadurch gekennzeichnet,**
**dass** der räumliche Versatz'zwischen der Hauptplatte (13) und dem Ausleger (15) durch das Verbindungselement (17) individuell einstellbar ist.

12. Implantat nach einem der vorhergehenden Ansprüche,
**dadurch gekennzeichnet,**
**dass** das Verbindungselement (17) an der Hauptplatte (13) und/oder am Ausleger (15) in unterschiedlichen Positionen fixierbar ist.

13. Implantat nach einem der vorhergehenden Ansprüche,
**dadurch gekennzeichnet,**
**dass** die Hauptplatte (13) wenigstens einen Durchgang (27) zum Hindurchführen des Verbindungselementes (17) aufweist.

14. Implantat nach Anspruch 13,
**dadurch gekennzeichnet,**
**dass** der Durchgang (27) sich im Wesentlichen parallel zu der durch die Hauptplatte (13) festgelegten Ebene erstreckt.

15. Implantat nach einem der vorhergehenden Ansprüche,
**dadurch gekennzeichnet,**
**dass** das Verbindungselement (17) eine langgestreckte Form aufweist.

16. Implantat nach einem der vorhergehenden Ansprüche,
**dadurch gekennzeichnet,**
**dass** das Verbindungselement (17) verbiegbar ist.

17. Implantat nach einem der vorhergehenden Ansprüche,
**dadurch gekennzeichnet,**
**dass** das Verbindungselement (17) ein Draht oder Faden ist.

18. Implantat nach einem der vorhergehenden Ansprüche,
**dadurch gekennzeichnet,**
**dass** das Verbindungselement (17) durch Anbinden, Verhaken und/oder Verrasten mit der Hauptplatte (13) und/oder dem Ausleger (15) koppelbar ist.

19. Implantat nach einem der vorhergehenden Ansprüche,
**dadurch gekennzeichnet,**
**dass** wenigstens zwei jeweils durch zumindest einen Durchgang (27) der Hauptplatte (13) hindurch geführte Verbindungselemente (17) auf der dem Ausleger (15) abgewandten Seite der Hauptplatte (13) insbesondere durch Verknoten oder Zusammendrehen ihrer freien Enden miteinander verbindbar sind.

20. Implantat nach einem der vorhergehenden Ansprüche,
**dadurch gekennzeichnet,**
**dass** der Ausleger (15) und/ oder das Verbindungselement (17) aus Metall, z.B. Titan, oder aus Kunststoff hergestellt sind.

21. Implantat nach einem der vorhergehenden Ansprüche,
**dadurch gekennzeichnet,**
**dass** die Hauptplatte (13) und/ oder der Ausleger (15) wenigstens einen haken- oder klauenartigen Fortsatz (29) aufweist.

22. Implantat nach einem der vorhergehenden Ansprüche,
**dadurch gekennzeichnet,**
**dass** unterschiedliche Implantatkonfigurationen aus Hauptplatte (13) und mit der Hauptplatte (13) verbundenem Ausleger (15) herstellbar sind, die bezüglich der Hauptplatte (13) und insbesondere bezüglich einer Längsachse der Hauptplatte (13) symmetrisch sind.

23. Implantat nach einem der vorhergehenden Ansprüche,
**dadurch gekennzeichnet,**
**dass** der Ausleger (15) und das Verbindungselement (17) separat hergestellt und derart fest miteinander verbunden sind, dass Ausleger (15) und Verbindungselement (17) während einer Operation als eine Einheit handhabbar sind.

24. Implantat nach Anspruch 23,
**dadurch gekennzeichnet,**
**dass** der Ausleger (15) und das Verbindungselement (17) unlösbar miteinander verbunden sind, insbesondere durch Verschweißen.

25. Implantat nach einem der vorhergehenden Ansprüche,
**dadurch gekennzeichnet,**
**dass** die Hauptplatte (13) und der Ausleger (15) über das Verbindungselement (17) einseitig miteinander verbunden sind.

26. Implantat nach einem der vorhergehenden Ansprüche,
**dadurch gekennzeichnet,**
**dass** der Ausleger (15) plattenförmig ausgebildet ist und eine geringere Dicke aufweist als die Hauptplatte (13), wobei insbesondere die Dicke des Auslegers (15) weniger als die Hälfte der Dicke der Hauptplatte (13) beträgt.

27. Implantat nach einem der vorhergehenden Ansprüche,
**dadurch gekennzeichnet,**
**dass** der Ausleger (15) während einer Operation werkzeuglos verformbar ist.

28. Implantat nach einem der vorhergehenden Ansprüche,
**dadurch gekennzeichnet,**
**dass** der Ausleger (15) eine kleinere Grundfläche aufweist als die Hauptplatte (13).

29. Implantat nach einem der vorhergehenden Ansprüche,
**dadurch gekennzeichnet,**
**dass** der Ausleger (15) eine für alle gängigen Frakturen eines bestimmten Knochens ausreichend große Grundform aufweist und zur Anpassung an eine jeweils zu versorgende Knochenfraktur in die erforderliche Form und Größe zuschneidbar ist.

30. Implantat nach einem der vorhergehenden Ansprüche,
**dadurch gekennzeichnet,**
**dass** der Ausleger (15) mit wenigstens fünf Durchgängen zur Aufnahme von Befestigungselementen (19), insbesondere von Knochenschrauben, versehen ist.

31. Implantat nach einem der vorhergehenden Ansprüche,
**dadurch gekennzeichnet,**
**dass** der Ausleger aus einem bioresorbierbaren Material, insbesondere aus einem Polymer, besteht.

32. Implantat nach Anspruch 31,
**dadurch gekennzeichnet,**
**dass** das bioresorbierbare Material bei Temperaturen zwischen 50 und 90 °C, insbesondere nach einem Bad in einer entsprechend warmen Salz-Lösung, plastisch verformbar ist.

33. Implantatsystem zur Versorgung von Knochenfrakturen, insbesondere von proximalen Humerusfrakturen, mit wenigstens einer am Knochen (11) fixierbaren Hauptplatte (13), wenigstens einem Ausleger (15) und einem Satz von flexiblen Verbindungselementen (17), über welche der Ausleger (15) mit der Hauptplatte (13) derart verbindbar ist, dass der Ausleger (15) räumlich versetzt zur Hauptplatte (13) am Knochen (11) fixierbar ist, wobei die Verbindungselemente (17) einsatzbereit vorgefertigt sind und sich hinsichtlich Form, Größe und/oder Länge voneinander unterscheiden.

34. Implantatsystem nach Anspruch 33,
**dadurch gekennzeichnet,**
**dass** zumindest eines der Verbindungselemente (17) eine U-Form aufweist und sowohl im Ausleger (15) als auch in der Hauptplatte (13) jeweils wenigstens ein Paar von insbesondere in Form von Bohrungen vorgesehenen Durchgängen (27, 139) für ein Verbindungselement (17) ausgebildet ist, deren Abstand dem der U-Schenkel des Verbindungselementes (17) entspricht.
